(19) Europäisches Patentamt
European Patent Office
Office européen des brevets

(11) Veröffentlichungsnummer: **0 369 234 A2**


# (12) EUROPÄISCHE PATENTANMELDUNG


(21) Anmeldenummer: **89120153.5**

(22) Anmeldetag: **31.10.89**

(51) Int. Cl.5: **C07D 285/125, C07D 417/12, A01N 43/82**

(30) Priorität: **12.11.88 DE 3838432**

(43) Veröffentlichungstag der Anmeldung: **23.05.90 Patentblatt 90/21**

(84) Benannte Vertragsstaaten: **BE CH DE ES FR GB GR IT LI NL**



(71) Anmelder: **BAYER AG**

**D-5090 Leverkusen 1 Bayerwerk(DE)**

(72) Erfinder: **Diehr, Hans-Joachim, Dr.**
**Höhe 35**
**D-5600 Wuppertal 11(DE)**
Erfinder: **Marhold, Albrecht, Dr.**
**Carl-Duisberg-Strasse 329**
**D-5090 Leverkusen 1(DE)**
Erfinder: **Brandes, Wilhelm, Dr.**
**Eichendorffstrasse 3**
**D-5653 Leichlingen(DE)**
Erfinder: **Hänssler, Gerd, Dr.**
**Am Arenzberg 58a**
**D-5090 Leverkusen 3(DE)**


## (54) 2,5-Disubstituierte 1,3,4-Thiadiazol-Derivate, ein Verfahren zu ihrer Herstellung und ihre Verwendung in Schädlingsbekämpfungsmitteln.


(57) 2,5-Disubstituierte 1,3,4-Thiadiazol-Derivate der Formel (I)

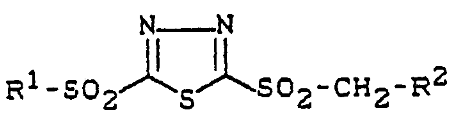

(I)

in welcher
$R^1$ für Alkyl oder unsubstituiertes oder substituiertes Aralkyl steht und
$R^2$ für jeweils unsubstituiertes oder substituiertes Aryl, Aralkyl oder für einen unsubstituierten oder substituierten und/oder gegebenenfalls benzoanellierten Heterocyclus steht - ausgenommen die Verbindung 2,5-Bis-[(phenylmethyl)-sulfonyl]-1,3,4-thiadiazol -,
und deren Verwendung zur Bekämpfung von Schädlingen, besonders von Pilzen.

Die neuen 2,5-disubstituierten 1,3,4-Thiadiazol-Derivate sind durch die Formel (I) allgemein definiert Die Verbindungen können nach Analogieverfahren z.B. aus geeigneten 2,5-Dimercapto-1,3,4-Thiadiazol-Derivaten mit geeigneten Oxidationsmitteln hergestellt werden.


EP 0 369 234 A2

## 2,5-Disubstituierte 1,3,4-Thiadiazol-Derivate, ein Verfahren zu ihrer Herstellung und ihre Verwendung in Schädlingsbekämpfungsmitteln

Die vorliegende Erfindung betrifft neue 2,5-disubstituierte 1,3,4-Thiadiazol-Derivate, ein Verfahren zu ihrer Herstellung und ihre Verwendung in Schädlingsbekämpfungsmitteln, vor allem als Fungizide.

Es ist bereits bekannt, daß bestimmte 1,3,4-Thiadiazole, wie z.B. 2,5-Bisalkylsulfonyl-1,3,4-thiadiazole, fungizide Eigenschaften besitzen (vgl. BE 716.257).

Außerdem ist u.a. die fungizide Wirkung von 2-Methylsulfonyl-5-thiocyanomethylsulfonyl-1,3,4-thiadiazol bekannt (vgl. Rec. Trav. Chim. Pays-Bas 90, No. 2, 97 - 104 (1971)).

Die Wirkung dieser Verbindungen ist jedoch insbesondere bei niedrigen Aufwandmengen und -konzentrationen nicht immer in allen Anwendungsbereichen völlig zufriedenstellend.

Außerdem ist 2,5-Dimethylsulfonyl-1,3-4-thiadiazol als Ausgangsverbindung für entsprechende Sulfonamide beschrieben (vgl. Abstr. Papers, Am. Chem. Soc. 1965, Nr. 150, 138).

Weiterhin ist die Verbindung 2,5-Bis[(phenylmethyl)-sulfonyl]-1,3,4-thiadiazol bekannt (vgl. Heterocyclus, 20, 19 - 22, 1983).

Es wurden neue 2,5-disubstituierte 1,3,4-Thiadiazol-Derivate der Formel (I)

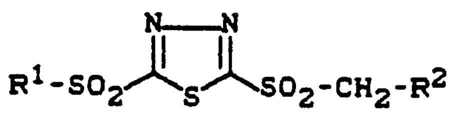

(I)

gefunden, in welcher
$R^1$ für Alkyl oder unsubstituiertes oder substituiertes Aralkyl steht und
$R^2$ für jeweils unsubstituiertes oder substituiertes Aryl, Aralkyl oder für einen unsubstituierten oder substituierten und/oder gegebenenfalls benzoanellierten Heterocyclus steht,
ausgenommen die Verbindung 2,5-Bis-[(phenylmethyl)-sulfonyl]-1,3,4-thiadiazol (vgl. Heterocyclus, 20, 19-22, 1983).

Weiterhin wurde gefunden, daß man die neuen 2,5-disubstituierten 1,3,4-Thiadiazol-Derivate der Formel (I)

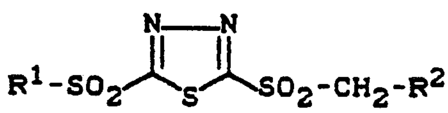

(I)

in welcher
$R^1$ für Alkyl oder unsubstituiertes oder substituiertes Aralkyl steht und
$R^2$ für jeweils unsubstituiertes oder substituiertes Aryl, Aralkyl oder für einen unsubstituierten oder substituierten und/oder gegebenenfalls benzoanellierten Heterocyclus steht,
erhält, wenn man
2,5-Dimercapto-1,3,4-thiadiazol-Derivate der Formel (II)

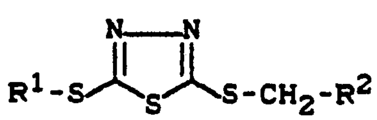

(II)

in welcher
$R^1$ und $R^2$ die oben angegebene Bedeutung haben,
mit einem Oxidationsmittel, gegebenenfalls in Gegenwart eines Verdünnungsmittels und gegebenenfalls in Gegenwart eines Katalysators, umsetzt.

Die erfindungsgemäßen 2,5-disubstituierten 1,3,4-Thiadiazol-Derivate zeigen eine sehr gute fungizide Wirksamkeit und stellen somit eine wertvolle Bereicherung der Technik dar.

In den allgemeinen Formeln bedeutet Alkyl im allgemeinen geradkettiges oder verzweigtes Alkyl mit 1 bis 8 Kohlenstoffatomen, bevorzugt mit 1 bis 6 und besonders bevorzugt mit 1 bis 4 Kohlenstoffatomen; beispielhaft seien genannt Methyl, Ethyl, n- und i-Propyl, n-, i-, sec.-und t-Butyl, Pentyl, Hexyl und Octyl.

Aryl steht im allgemeinen als solches oder in Zusammensetzungen wie Aralkyl im allgemeinen für einen

aromatischen Rest mit 6 bis 10 Kohlenstoffatomen. Arylreste sind bevorzugt Phenyl oder Naphthyl, ganz besonders bevorzugt Phenyl.

Aralkyl steht im allgemeinen für im Arylteil mit 6 bis 10 und im Alkylteil mit 1 bis 6, vorzugsweise 1 bis 5 Kohlenstoffatomen in der Definition von $R^1$ und 1 bis 4 Kohlenstoffatomen in der Definition von $R^2$, besonders bevorzugt mit 1 bis 3 bzw 1 oder 2 Kohlenstoffatomen im Alkylteil. Beispielhaft seien genannt Phenylmethyl, Phenethyl, Naphthylmethyl und Naphthylethyl.

Heteroaromatischer Ring steht im allgemeinen für einen 5- oder 6-gliedrigen Ring, der ein oder mehrere Heteroatome, bevorzugt 1 bis 3 gleiche oder verschiedene Heteroatome, enthält und die entsprechenden benzoanellierten Ringe.

Als Heteroatome seien vorzugsweise Sauerstoff, Schwefel und Stickstoff genannt; beispielhaft seien genannt: Pyridyl, Pyrimidyl, Pyridazyl, Thienyl, Furyl, Pyrazolyl, Thiadiazolyl, Chinolyl, Isochinolyl, Benzoxazolyl, Benzthiazolyl, Benzimidazolyl und 1,2,4-Oxadiazolyl.

Die Substituenten für die Arylreste als solche oder in Zusammensetzungen wie Arylalkyl und für die heterocyclischen Ringe haben die im folgenden angegebenen Bedeutungen.

Halogen steht im allgemeinen als Substituent der Reste für Fluor, Chlor, Brom und Jod, bevorzugt für Fluor, Chlor und Brom, besonders bevorzugt für Fluor und Chlor.

Alkyl steht im allgemeinen als Substituent der Reste für geradkettiges oder verzweigtes Alkyl, bevorzugt mit 1 bis 6, besonders bevorzugt mit 1 bis 4 Kohlenstoffatomen, ganz besonders bevorzugt sind Methyl, Ethyl und t-Butyl. Die beispielhafte Aufzählung entspricht der weiter oben gegebenen.

Alkoxy steht im allgemeinen als Substituent der Reste für geradkettiges oder verzweigtes Alkoxy mit 1 bis 6, besonders bevorzugt 1 bis 3 Kohlenstoffatomen; beispielhaft seien genannt: Methoxy, Ethoxy, n- und i-Propoxy, n-, i-, sec.- und t-Butoxy, n-Hexoxy und i-Hexoxy.

Alkylsulfonyl steht im allgemeinen als Substituent der Reste für geradkettiges oder verzweigtes Alkylsulfonyl mit 1 bis 4, besonders bevorzugt mit 1 bis 3 Kohlenstoffatomen, ganz besonders bevorzugt mit 1 Kohlenstoffatom; beispielhaft seien genannt: Methylsulfonyl, Ethylsulfonyl, n-, i-, sec.- und t-Butylsulfonyl.

Halogenalkyl Halogenalkoxy, Halogenalkylsulfonyl stehen im allgemeinen als Substituenten in den Resten für geradkettige oder verzweigte Reste mit je 1 bis 4 Kohlenstoffatomen, besonders bevorzugt mit 1 oder 2 Kohlenstoffatomen und jeweils 1 bis 5 gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy, Trifluorchlorethoxy und Fluormethylsulfonyl, Chlormethylsulfonyl, Bromethylsulfonyl, Fluorethylsulfonyl, Chlorethylsulfonyl, Brommethylsulfonyl, Fluorpropylsulfonyl, Chlorpropylsulfonyl, Brompropylsulfonyl, Fluorbutylsulfonyl, Chlorbutylsulfonyl, Fluor-isopropylsulfonyl, Chlor-isopropylsulfonyl, Chlorbutylsulfonyl, Difluormethylsulfonyl, Trifluormethylsulfonyl, Dichlormethylsulfonyl, Trichlormethylsulfonyl, Difluorethylsulfonyl, Trifluorethylsulfonyl, Tetrafluorethylsulfonyl, Trichlorethylsulfonyl, Chlordifluormethylsulfonyl und Trifluorchlorethylsulfonyl, besonders hervorzuheben sind Trifluormethyl, Trifluormethoxy, Trifluormethylsulfonyl.

Alkoxycarbonyl steht im allgemeinen als Substituent in den Resten für geradkettiges oder verzweigtes Alkoxycarbonyl mit 1 bis 4, vorzugsweise 1 oder 2 Kohlenstoffatomen im Alkoxyrest; beispielhaft seien genannt: Methoxycarbonyl, Ethoxycarbonyl, n-Propoxycarbonyl, i-Propoxycarbonyl, n-, i-, sec.- und t-Butoxycarbonyl.

Einfach bis mehrfache Substitution bedeutet 1- bis 5-fach, besonders bevorzugt 1- bis 3-fach bzw. 1- oder 2-fach substituiert, wenn nicht anders angegeben.

Die erfindungsgemäßen 2,5-disubstituierten 1,3,4-Thiadiazol-Derivate sind durch die Formel (I) allgemein definiert. Bevorzugt werden diejenigen Verbindungen der Formel (I), in welcher

$R^1$ für geradkettiges oder verzweigtes Alkyl mit 1 bis 6 Kohlenstoffatomen oder unsubstituiertes oder einfach bis mehrfach, gleich oder verschieden substituiertes Aralkyl mit 1 bis 5 Kohlenstoffatomen im Alkylteil und 6 bis 10 Kohlenstoffatomen im Arylteil steht, wobei als Arylsubstituenten infrage kommen: Halogen, Nitro, Cyano, Alkyl oder Alkoxy mit jeweils 1 bis 6 Kohlenstoffatomen, Halogenalkyl, Halogenalkoxy, Alkylsulfonyl, Halogenalkylsul fonyl oder Alkoxycarbonyl mit jeweils 1 bis 4 Kohlenstoffatomen in den einzelnen Alkyl- bzw. Alkoxyteilen und im Fall des Halogenalkyls, Halogenalkoxys und Halogenalkylsulfonyls mit 1 bis 5 gleichen oder verschiedenen Halogenatomen und

$R^2$ für jeweils unsubstituiertes oder einfach bis mehrfach, gleich oder verschieden substituiertes Aryl oder

Aralkyl mit 6 bis 10 Kohlenstoffatomen im Arylteil und im Fall des Aralkyls mit 1 bis 4 Kohlenstoffatomen im Alkylteil steht, wobei als Arylsubstituenten jeweils infrage kommen: Halogen, Nitro, Cyano, Alkyl und Alkoxy mit jeweils 1 bis 6 Kohlenstoffatomen und Halogenalkyl, Halogenalkoxy, Alkylsulfonyl, Halogenalkylsulfonyl oder Alkoxycarbonyl mit jeweils 1 bis 4 Kohlenstoffatomen in den einzelnen Alkyl- bzw. Alkoxyteilen und im Fall des Halogenalkyls, Halogenalkoxys und Halogenalkylsulfonyls mit 1 bis 5 gleichen oder verschiedenen Halogenatomen; für einen unsubstituierten oder einfach bis mehrfach, gleich oder verschieden substituierten, gegebenenfalls benzoanellierten hetero-aromatischen 5- oder 6-gliedrigen Ring, der gleiche oder verschiedene, ein oder mehrere Heteroatome wie Sauerstoff, Schwefel und Stickstoff enthält, steht, wobei als Substituenten Halogen und Alkyl mit 1 bis 6 Kohlenstoffatomen infrage kommen, ausgenommen die Verbindung 2,5-Bis-[(phenylmethyl)-sulfonyl]-1,3,4-thiadiazol.

Besonders bevorzugt sind Verbindungen der Formel (I), in welcher

$R^1$ für geradkettiges oder verzweigtes Alkyl mit 1 bis 4 Kohlenstoffatomen, für unsubstituiertes oder einfach bis dreifach, gleich oder verschieden substituiertes Phenylalkyl oder Naphthylalkyl mit 1 bis 3 Kohlenstoffatomen in den Alkylteilen steht, wobei als Substituenten genannt seien: Halogen, Nitro, Cyano, Alkyl mit 1 bis 4 Kohlenstoffatomen, Alkoxy mit 1 bis 3 Kohlenstoffatomen, Alkylsulfonyl mit 1 bis 3 Kohlenstoffatomen, Halogenalkyl, Halogenalkoxy und Halogenalkylsulfonyl mit jeweils 1 oder 2 Kohlenstoffatomen und 1 bis 5 gleichen oder verschiedenen Halogenatomen und Alkoxycarbonyl mit 1 oder 2 Kohlenstoffatomen im Alkoxyteil und

$R^2$ für unsubstituiertes oder einfach bis dreifach, gleich oder verschieden substituiertes Phenyl, Naphthyl, Phenylalkyl oder Naphthylalkyl mit 1 oder 2 Kohlenstoffatomen je Alkylteil steht, wobei als Substituenten der Ringe genannt seien: Halogen, Nitro, Cyano, Alkyl mit 1 bis 4 Kohlenstoffatomen, Alkoxy mit 1 bis 3 Kohlenstoffatomen, Alkylsulfonyl mit 1 bis 3 Kohlenstoffatomen, Halogenalkyl, Halogenalkoxy und Halogenalkylsulfonyl mit jeweils 1 oder 2 Kohlenstoffatomen und 1 bis 5 gleichen oder verschiedenen Halogenatomen und Alkoxycarbonyl mit 1 oder 2 Kohlenstoffatomen im Alkoxyteil; für einen unsubstituierten oder einfach bis dreifach, gleich oder verschieden substituierten, gegebenenfalls benzoanellierten hetero-aromatischen 5- oder 6-gliedrigen Ring steht, der 1, 2 oder 3 gleiche oder verschiedene Heteroatome, z.B. Sauerstoff, Schwefel und Stickstoff, enthält. Als Substituenten für den Heterocyclus seien Halogen und geradkettiges oder verzweigtes Alkyl mit 1 bis 4 Kohlenstoffatomen genannt, ausgenommen die Verbindung 2,5-Bis-[(phenyl-methyl)-sulfonyl]-1,3,4-thiadiazol.

Ganz besonders bevorzugt sind Verbindungen der Formel (I), in welcher

$R^1$ für Methyl, Ethyl, n- und i-Propyl, n-, i-, s- und t-Butyl oder unsubstituiertes oder einfach bis dreifach, gleich oder verschieden substituiertes Benzyl steht, wobei als Substituenten infrage kommen: Fluor, Chlor, Brom, Nitro, Cyano, Methyl, Ethyl, t-Butyl, Methoxy, Trifluormethyl, Trifluormethoxy, Trifluormethylsulfonyl, Methoxycarbonyl, und Ethoxycarbonyl und

$R^2$ für jeweils unsubstituiertes oder einfach bis dreifach, gleich oder verschieden substituiertes Phenyl, Naphthyl, Benzyl oder Phenethyl steht, wobei als Substituenten jeweils infrage kommen: Fluor, Chlor, Brom, Nitro, Cyano, Methyl, Ethyl, t-Butyl, Methoxy, Ethoxy, Trifluormethyl, Trifluormethoxy, Methylsulfonyl, Trifluormethylsulfonyl, Methoxycarbonyl oder Ethoxycarbonyl; weiter für jeweils unsubstituiertes oder ein- oder zweifach, gleich oder verschieden durch Fluor, Chlor, Brom, Methyl oder Ethyl substituiertes Pyridyl, Benzoxazolyl, Benzthiazolyl, Benzimidazolyl, 1,3-Thiazolyl oder 1,2,4-Oxadiazolyl steht,
ausgenommen die Verbindung 2,5-Bis-[(phenylmethyl)-sulfonyl]-1,3,4-thiazol.

Verwendet man als Ausgangsstoffe beispielsweise 2-Methylmercapto-5-(4-chlorbenzylmercapto)-1,3,4-thiadizol, Natriumwolframat als Katalysator und zur Oxidation Wasserstoffperoxid, so läßt sich der Reaktionsablauf des erfindungsgemäßen Verfahrens durch das folgende Formelschema darstellen:

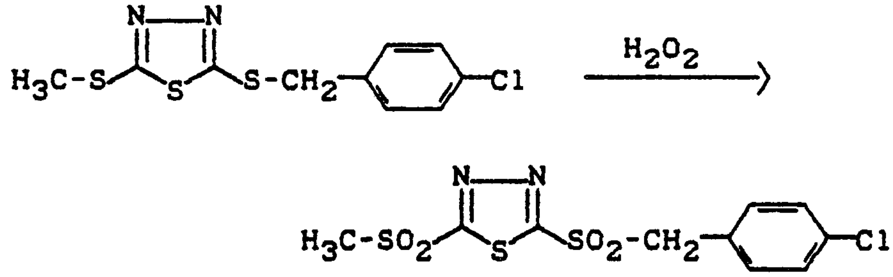


Die zur Durchführung des erfindungsgemäßen Verfahrens als Ausgangsstoffe benötigten 2,5-Dimercapto-1,3,4-thiadiazol-Derivate sind durch die Formel (II) allgemein definiert. In dieser Formel (II) stehen $R^1$ und $R^2$ vorzugsweise bzw. insbesondere für diejenigen Substituenten, die oben bei der

Beschreibung der neuen 2,5-disubstituierten 1,3,4-Thiadiazol-Derivate der Formel (I) als bevorzugt bzw. besonders bevorzugt für diese Reste genannt wurden.

Die 2,5-Dimercapto-1,3,4-thiadiazol-Derivate der Formel (II) sind teilweise bekannt und/oder können nach bekannten Verfahren in einfacher, analoger Weise hergestellt werden (vgl. z.B. EP-PS 214 732 und JP 87 039583), indem man beispielsweise

(A) 2-substituierte 1,3,4-Thiadiazol-Derivate der Formel (III)

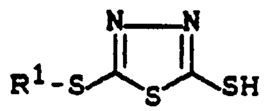

(III)

oder deren Alkalimetallsalze
in welcher
$R^1$ die oben angegebene Bedeutung hat
mit Halogeniden der Formel (IV)
$X-CH_2-R^2$ (IV)
in welcher
$R^2$ die oben angegebene Bedeutung hat und
X für Halogen, insbesondere Chlor oder Brom steht,
gegebenenfalls in Gegenwart eines Verdünnungsmittels, wie beispielsweise Aceton, Methylethylketon oder Acetonitril und gegebenenfalls in Gegenwart einer Base, wie beispielsweise Kaliumcarbonat, bei Temperaturen zwischen 20° C und 100° C umsetzt
oder

(B) 5-substituierte 1,3,4-Thiadiazole der Formel (V)

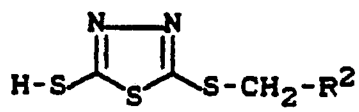

(V)

oder deren Alkalimetallsalze
in welcher
$R^2$ die oben angegebene Bedeutung hat
mit Halogeniden der Formel (VI)
$R^1 - X$ (VI)
in welcher
$R^1$ die oben angegebene Bedeutung hat und
X für Halogen, insbesondere Chlor oder Brom steht,
gegebenenfalls in Gegenwart eines Verdünnungsmittels, wie beispielsweise Aceton, Methylethylketon oder Acetonitril und gegebenenfalls in Gegenwart einer Base, wie beispielsweise Kaliumcarbonat, bei Temperaturen zwischen 20° C und 100° C umsetzt.

Die als Ausgangsstoffe benötigten Verbindungen der Formel (III) sind bekannt (vgl. DE-OS 24 59 672).

Die als Ausgangsstoffe benötigten Verbindungen der Formel (V) sind bekannt (vgl. z.B. EP-PS 214 732 und JP 87039583).

Die außerdem als Ausgangsstoffe benötigten Halogenide der Formeln (IV) und (VI) sind allgemein bekannte Verbindungen der organischen Chemie.

Die Oxidationsmittel für das erfindungsgemäße Verfahren sind an sich bekannt (vergl. Reaktionen der organischen Synthese, G. Thieme Verlag, Stuttgart (1978), S. 472).

Beispielsweise seien Wasserstoffperoxid, Perameisensäure, Peressigsäure, Perbenzoesäure, m-Chlorperbenzoesäure und/oder Natriummetaperiodat und wässrige Lösungen von Halogen, insbesondere Chlor oder Brom genannt (vgl. S. Oae, Organic Chemistry of Sulfur, Plenum Press, New York, 1977, S.529ff) genannt.

Bevorzugte Oxidationsmittel sind Wasserstoffperoxid und/oder Persäuren.

Die Menge an Oxidationsmittel beträgt im allgemeinen etwa 1 bis 4 Äquivalente, bezogen auf die 2,5-Dimercapto-1,3,4-thiadiazol-Derivate der Formel (II).

Als Verdünnungsmittel für die Oxidationsreaktion kommen alle inerten organischen Lösungsmittel in Frage, die sich unter den Oxidationsbedingungen nicht verändern.

Hierzu gehören vorzugsweise chlorierte Kohlenwasserstoffe, wie Methylenchlorid, Chloroform, Tetrachlorkohlenstoff, 1,2-Dichlorethan, 1,2,2-Trichlorethan und Chlorbenzol; Alkohole, vorzugsweise Methanol, Ethanol oder Isopropanol; niedere Fettsäuren, bevorzugt Ameisensäure, Essigsäure oder Propionsäure. Das erfindungsgemäße Verfahren kann auch in Mischung der Lösungsmittel mit Wasser oder in Gemischen von mehreren Lösungsmitteln durchgeführt werden.

Als Katalysator bei der Oxidation können die üblicherweise hierfür verwendeten Salze von Metallen der IV., V. und VI. Nebengruppe des Periodensystems der Elemente verwendet werden, insbesondere seien Vanadiumpentoxid, Natriummetavanadat, Natriummolybdat und/oder Natriumwolframat genannt.

Die günstigste Menge an Oxidationsmittel und Katalysator bei der Oxidation kann ebenfalls leicht durch Vorversuche ermittelt werden. Üblicherweise wird die oben angegebene Menge des Oxidationsmittels mit etwa 0,01 bis 0,1, bevorzugt 0,015 bis 0,09, Mol Katalysator je Mol Mercaptoverbindung der Formel (IV) eingesetzt. Die Verdünnungsmittelmenge ist nicht kritisch und kann ebenfalls leicht durch Vorversuche ermittelt werden.

Die Oxidation wird im allgemeinen im Temperaturbereich von ca. -60 °C bis +120 °C, vorzugsweise im Temperaturbereich von -60 °C bis +80 °C durchgeführt.

Die Oxidation kann bei Normaldruck, aber auch bei einem Unter- oder Überdruck (beispielsweise im Druckbereich von 0,5 bis 1,5 bar) durchgeführt werden. Bevorzugt führt man die Oxidation bei Normaldruck aus.

Bei der Herstellung der erfindungsgemäßen 2,5-disubstituierten 1,3,4-Thiadiazol-Derivate der Formel (I) nach dem erfindungsgemäßen Verfahren setzt man im allgemeinen jeweils die äquivalente Menge an Oxidationsmittel ein, d. h. man setzt pro Mol 2,5-Dimercapto-1,3,4-thiadiazol der Formel (II) vier Oxidationsäquivalente, gegebenenfalls auch einen größeren Überschuß, ein.

Die Isolierung der neuen 2,5-disubstituierten 1,3,4-Thiadiazol-Derivate der allgemeinen Formel (I) erfolgt in allgemein üblicher Art und Weise, beispielsweise durch Versetzen des Reaktionsgemisches mit Wasser, Extraktion mit einem organischen Lösungsmittel, Einengen und gegebenenfalls Umkristallisieren.

Die erfindungsgemäßen Wirkstoffe weisen eine starke biologische Wirkung auf und können zur Bekämpfung von unerwünschten Schädlingen praktisch eingesetzt werden. Die Wirkstoffe sind z.B.für den Gebrauch als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;

Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg protektiv zur Bekämpfung von Pyricularia-Arten an Reis, Plasmopara-Arten an Reben und Venturia-Arten an Äpfeln eingesetzt werden.

Außerdem besitzen einige der erfindungsgemäßen Wirkstoffe eine gute bakterizide Wirkung sowie eine gute fungizide Wirkung gegen Pellicularia-Arten, wie beispielsweise Pellicularia sasakii.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den

Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew-%, vorzugsweise von 0,0001 bis 0,02 % am Wirkungsort erforderlich.

Die Herstellung und die Verwendung der erfindungsgemäßen Wirkstoffe geht aus den folgenden Beispielen hervor.

Herstellungsbeispiele

Beispiel 1:

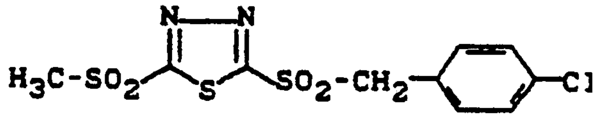

10 g (0,035 Mol) 2-Methylmercapto-5-(4-chlorbenzylmercapto)-1,3,4-thiadiazol und 1 g Natriumwolframat werden in 100 ml Ameisensäure langsam mit 40 ml 35 %iger Wasserstoffperoxid-Lösung versetzt, wobei die Innentemperatur bis auf 70°C ansteigt. Das Reaktionsgemisch wird nach dem Zutropfen 30 Minuten bei 70°C nachgerührt, dann auf Raumtemperatur abgekühlt und mit Wasser verdünnt. Das ausgefallene Produkt wird abgesaugt und getrocknet.

Man erhält 11,1 g (89,8 % der Theorie) 2-Methylsulfonyl-5-(4-chlorbenzylsulfonyl)-1,3,4-thiadiazol als kristallinen weißen Feststoff vom Schmelzpunkt 159-160°C.

In analoger Weise zu Beispiel 1 und unter Berücksichtigung der Angaben in der Beschreibung zu dem erfindungsgemäßen Verfahren, werden die nachfolgend in Tabelle 1 aufgeführten Endprodukte der Formel (I)

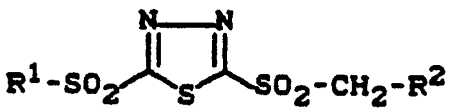

(I)

erhalten:

Tabelle 1

| Bsp. Nr. | $R^1$ | $R^2$ | Schmelz-punkt (°C) |
|---|---|---|---|
| 2 | $CH_3$ | 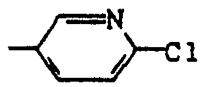 | 189 |
| 3 | $CH_3$ | 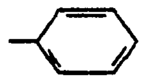 | 138 |
| 4 | $CH_3$ | 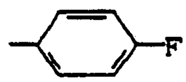 | 173 |
| 5 | $CH_3$ | 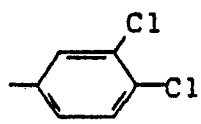 | 205-6 |
| 6 | $CH_3$ | 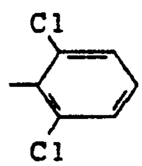 | 173 |
| 7 | $CH_3$ | 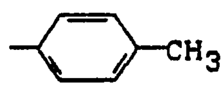 | 146 |
| 8 | $CH_3$ | 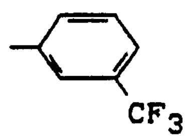 | 150 |
| 9 | $CH_3$ | 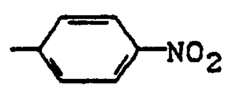 | 230-2 (Zers.) |
| 10 | $CH_3$ | 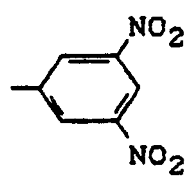 | 184-5 |

Tabelle 1 - Fortsetzung

| Bsp. Nr. | $R^1$ | $R^2$ | Schmelzpunkt (°C) |
|---|---|---|---|
| 11 | 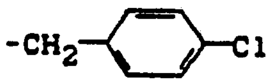 | 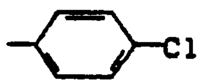 | 228 |
| 12 | $CH_3$ | 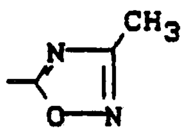 | 144-45 |
| 13 | $-C(CH_3)_3$ | 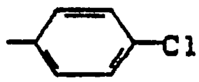 | 139 |
| 14 | $CH_3$ | 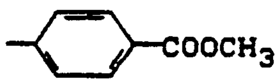 | 177 |
| 15 | $CH_3$ | 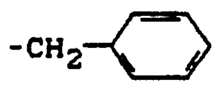 | 105 |
| 16 | $CH_3$ | 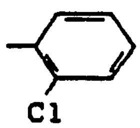 | 150 |
| 17 | $CH_3$ | 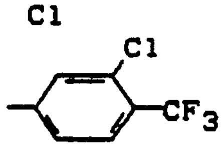 | 182-3 |
| 18 | $CH_3$ | 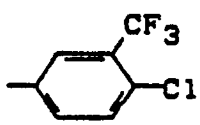 | 199-200 |
| 19 | $CH_3$ | 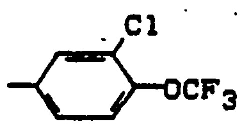 | 181 |
| 20 | $CH_3$ | 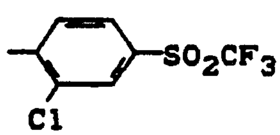 | 197 |

<u>Tabelle 1</u> - Fortsetzung

| Bsp. Nr. | $R^1$ | $R^2$ | Schmelzpunkt |
|---|---|---|---|
| 21 | $CH_3$ | 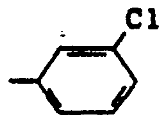 | 152 |
| 22 | $CH_3$ | 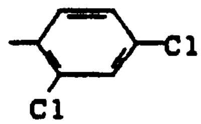 | 178 |
| 23 | $CH_3$ | 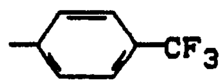 | 202 |
| 24 | $-CH(CH_3)_2$ | 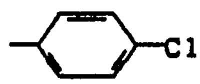 | 143 |
| 25 | $C_2H_5$ | 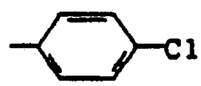 | 131 |
| 26 | $CH_3$ | 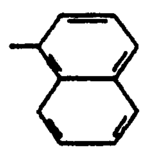 | 160-1 |
| 27 | $CH_3$ | 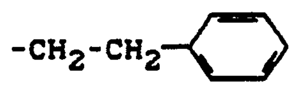 | 118-9 |
| 28 | $CH_3$ | 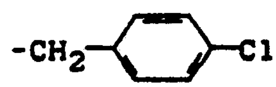 | 140 |
| 29 | $CH_3$ | 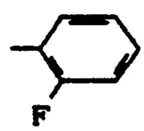 | 151 |
| 30 | $CH_3$ | 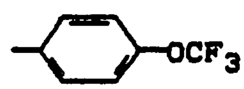 | 178-80 |

Tabelle 1 - Fortsetzung

| Bsp. Nr. | $R^1$ | $R^2$ | Schmelzpunkt |
|---|---|---|---|
| 31 | $CH_3$ | 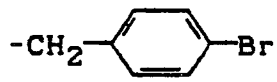 | 185-6 |
| 32 | $-C(CH_3)_3$ | 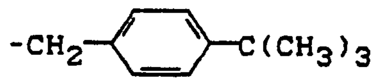 | 196-7 |
| 33 | $CH_3$ | 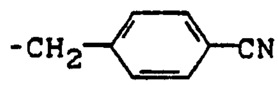 | 202-3 |
| 34 | $CH_3$ | 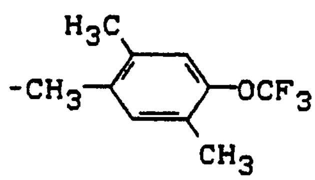 | 154-5 |
| 35 | $CH_3$ | 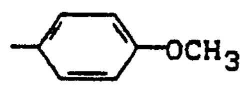 | 145 |
| 36 | $CH_3$ | 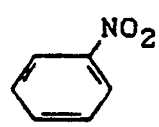 | 189-90 |
| 37 | $CH_3$ | 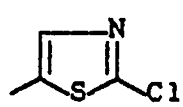 | 137 |
| 38 | $CH_3$ | 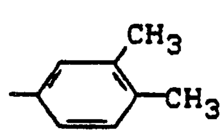 | 119-23 |

Herstellung der Ausgangsstoffe

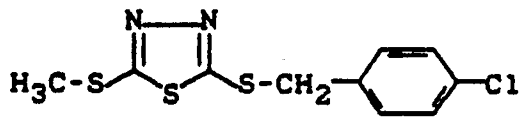

198 g (1,5 Mol) 2-Methylmercapto-5-mercapto-1,3,4-thiadiazol und 240 g (1,74 Mol) Kaliumcarbonat werden 30 Minuten in 2,2 l Acetonitril auf 40-50 °C erwärmt. Bei dieser Temperatur wird dann eine Lösung von 241 g (1,5 Mol) 4-Chlorbenzylchlorid in 500 ml Acetonitril eingetropft. Das Reaktionsgemisch wird eine Stunde bei 60 -70 °C nachgerührt, dann heiß abgesaugt und der Rückstand mit warmem Acetonitril nachgewaschen. Das Reaktionsprodukt kristallisiert beim Abkühlen aus.
Man erhält 389,5 g (90 % der Theorie) 2-Methylmercapto-5-(4-chlorbenzylmercapto)-1,3,4-thiadiazol vom Schmelzpunkt 77-78 °C.

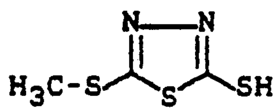

Zu einer Lösung von 61 g (0,5 Mol) Dithiocarbazinsäuremethylester in 100 ml Pyridin werden bei 40 - 50 °C 45,7 g = 36 ml (0,6 Mol) Schwefelkohlenstoff getropft und der entstehende Schwefelwasserstoff in Natronlauge geleitet. Nach Beendigung des Eintropfens wird das Reaktionsgemisch 30 Minuten auf 100 °C erhitzt, der restliche Schwefelwasserstoff mit Stickstoff ausgeblasen und das Lösungsmittel abdestilliert. Der Rückstand wird mit Wasser aufgenommen und abgesaugt.
Man erhält 71,6 g (87 % der Theorie) 2-Methylmercapto-5-mercapto-1,3,4-thiadiazol vom Schmelzpunkt 136 °C.

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

In diesem Test zeigen z.B. die erfindungsgemäßen Stoffe (1), (2), (5), (6) und (8) bei einer Wirkstoffkonzentration von 0,025 Gew.-% in der Spritzbrühe eine gute Wirkung.

Beispiel B

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

In diesem Test zeigen z.B. die erfindunsggemäßen Verbindungen (1), (3), (4), (6), (7) und (8) einen Wirkungsgrad von etwa 90 % bei einer Wirkstoffkonzentration von 10 ppm.

Beispiel C

Venturia-Test (Apfel) /protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigen z.B. die erfindunsggemäßen Verbindungen (1), (3), (5), (6), (7) und (8) einen Wirkungsgrad von etwa 85 % bei einer Wirkstoffkonzentration von 5 ppm.

**Ansprüche**

1. 2,5-Disubstituierte 1,3,4-Thiadiazol-Derivate der Formel (I)

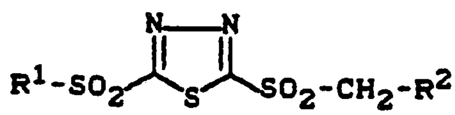

(I)

in welcher
$R^1$ für Alkyl oder unsubstituiertes oder substituiertes Aralkyl steht und
$R^2$ für jeweils unsubstituiertes oder substituiertes Aryl, Aralkyl oder für einen unsubstituierten oder substituierten und gegebenenfalls benzoanellierten Heterocyclus steht,
ausgenommen die Verbindung 2,5-Bis-[(phenylmethyl)-sulfonyl]-1,3,4-thiadiazol.

2. 2,5-Disubstituierte 1,3,4-Thiadiazol-Derivate gemäß Anspruch 1, worin in der Formel (I)
$R^1$ für geradkettiges oder verzweigtes Alkyl mit 1 bis 6 Kohlenstoffatomen oder unsubstituiertes oder einfach bis mehrfach, gleich oder verschieden substituiertes Aralkyl mit 1 bis 5 Kohlenstoffatomen im Alkylteil und 6 bis 10 Kohlenstoffatomen im Arylteil steht, wobei als Arylsubstituenten infrage kommen: Halogen, Nitro, Cyano, Alkyl oder Alkoxy mit jeweils 1 bis 6 Kohlenstoffatomen, Halogenalkyl, Halogenalkoxy, Alkylsulfonyl, Halogenalkylsulfonyl oder Alkoxycarbonyl mit jeweils 1 bis 4 Kohlenstoffatomen in den einzelnen Alkyl-bzw. Alkoxyteilen und im Fall des Halogenalkyls, Halogenalkoxys und Halogenalkylsulfonyls mit 1 bis 5 gleichen oder verschiedenen Halogenatomen und
$R^2$ für jeweils unsubstituiertes oder einfach bis mehrfach, gleich oder verschieden substituiertes Aryl oder Aralkyl mit 6 bis 10 Kohlenstoffatomen im Arylteil und im Fall des Aralkyls mit 1 bis 4 Kohlenstoffatomen im Alkylteil steht, wobei als Arylsubstituenten jeweils infrage kommen: Halogen, Nitro, Cyano, Alkyl und Alkoxy mit jeweils 1 bis 6 Kohlenstoffatomen und Halogenalkyl, Halogenalkoxy, Alkylsulfonyl, Halogenalkylsulfonyl oder Alkoxycarbonyl mit jeweils 1 bis 4 Kohlenstoffatomen in den einzelnen Alkyl- bzw. Alkoxyteilen und im Fall des Halogenalkyls, Halogenalkoxys und Halogenalkylsulfonyls mit 1 bis 5 gleichen oder verschiedenen

Halogenatomen; für einen unsubstituierten oder einfach bis mehrfach, gleich oder verschieden substituierten, gegebenenfalls benzoanellierten heteroaromatischen 5-oder 6-gliedrigen Ring, der gleiche oder verschiedene, ein oder mehrere Heteroatome enthält, steht, wobei als Substituenten Halogen und Alkyl mit 1 bis 6 Kohlenstoffatomen infrage kommen, ausgenommen die Verbindung 2,5-Bis-[(phenylmethyl)-sulfonyl]-1,3,4-thiadiazol.

3. 2,5-Disubstituierte 1,3,4-Thiadiazol-Derivate gemäß Anspruch 1, worin in der Formel (I)
$R^1$ für geradkettiges oder verzweigtes Alkyl mit 1 bis 4 Kohlenstoffatomen, für unsubstituiertes oder einfach bis dreifach, gleich oder verschieden substituiertes Phenylalkyl oder Naphthylalkyl mit 1 bis 3 Kohlenstoffatomen in den Alkylteilen steht, wobei als Substituenten genannt seien: Halogen, Nitro, Cyano, Alkyl mit 1 bis 4 Kohlenstoffatomen, Alkoxy mit 1 bis 3 Kohlenstoffatomen, Alkylsulfonyl mit 1 bis 3 Kohlenstoffatomen, Halogenalkyl, Halogenalkoxy und Halogenalkylsulfonyl mit jeweils 1 oder 2 Kohlenstoffatomen und 1 bis 5 gleichen oder verschiedenen Halogenatomen und Alkoxycarbonyl mit 1 oder 2 Kohlenstoffatomen im Alkoxyteil und
$R^2$ für unsubstituiertes oder einfach bis dreifach, gleich oder verschieden substituiertes Phenyl, Naphthyl, Phenylalkyl oder Naphthylalkyl mit 1 oder 2 Kohlenstoffatomen je Alkylteil steht, wobei als Substituenten der Ringe genannt seien: Halogen, Nitro, Cyano, Alkyl mit 1 bis 4 Kohlenstoffatomen, Alkoxy mit 1 bis 3 Kohlenstoffatomen, Alkylsulfonyl mit 1 bis 3 Kohlenstoffatomen, Halogenalkyl, Halogenalkoxy und Halogenalkylsulfonyl mit jeweils 1 oder 2 Kohlenstoffatomen und 1 bis 5 gleichen oder verschiedenen Halogenatomen und Alkoxycarbonyl mit 1 oder 2 Kohlenstoffatomen im Alkoxyteil; für einen unsubstituierten oder einfach bis dreifach, gleich oder verschieden substituierten, gegebenenfalls benzoanellierten heteroaromatischen 5- oder 6-gliedrigen Ring steht, der 1 bis 3 gleiche oder verschiedene Heteroatome, enthält. Als Substituenten für den Heterocyclus seien Halogen und geradkettiges oder verzweigtes Alkyl mit 1 bis 4 Kohlenstoffatomen genannt,
ausgenommen die Verbindung 2,5-Bis-[(phenylmethyl)-sulfonyl]-1,3,4-thiadiazol.

4. 2,5-Disubstituierte 1,3,4-Thiadiazol-Derivate gemäß Anspruch 1, worin in der Formel (I)
$R^1$ für Methyl, Ethyl, n- und i-Propyl, n-, i-, s- und t-Butyl oder unsubstituiertes oder einfach bis dreifach, gleich oder verschieden substituiertes Benzyl steht, wobei als Substituenten infrage kommen: Fluor, Chlor, Brom, Nitro, Cyano, Methyl, Ethyl, t-Butyl, Methoxy, Trifluormethyl, Trifluormethoxy, Trifluormethylsulfonyl, Methoxycarbonyl und Ethoxycarbonyl
und
$R^2$ für jeweils unsubstituiertes oder einfach bis dreifach, gleich oder verschieden substituiertes Phenyl, Naphthyl, Benzyl oder Phenethyl steht, wobei als Substituenten jeweils infrage kommen: Fluor, Chlor, Brom, Nitro, Cyano, Methyl, Ethyl, t-Butyl, Methoxy, Ethoxy, Tri fluormethyl, Trifluormethoxy, Methylsulfonyl, Trifluormethylsulfonyl, Methoxycarbonyl oder Ethoxycarbonyl; weiter für jeweils unsubstituiertes oder ein- oder zweifach, gleich oder verschieden durch Fluor, Chlor, Brom, Methyl oder Ethyl substituiertes Pyridyl, Benzoxazolyl, Benzthiazolyl, Benzimidazolyl, 1,3-Thiazolyl oder 1,2,4-Oxadiazolyl steht,
ausgenommen die Verbindung 2,5-Bis-[(phenylmethyl)-sulfonyl]-1,3,4-thiazol.

5. Verfahren zur Herstellung von 2,5-disubstituierten 1,3,4-Thiadiazol-Derivaten der Formel (I)

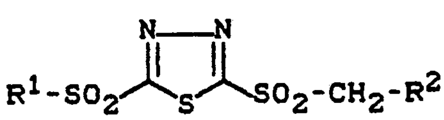
(I)

in welcher
$R^1$ für Alkyl oder unsubstituiertes oder substituiertes Aralkyl steht und
$R^2$ für jeweils unsubstituiertes oder substituiertes Aryl, Aralkyl oder für einen unsubstituierten oder substituierten und gegebenenfalls benzoanellierten Heterocyclus steht,
ausgenommen die Verbindung 2,5-Bis-[(phenylmethyl)-sulfonyl]-1,3,4-thiadiazol , dadurch gekennzeichnet, daß man
2,5-Dimercapto-1,3,4-thiadiazol-Derivate der Formel (II)

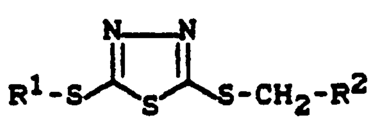
(II)

in welcher
$R^1$ und $R^2$ die oben angegebene Bedeutung haben,

mit einem Oxidationsmittel, gegebenenfalls in Gegenwart eines Verdünnungsmittels und gegebenenfalls in Gegenwart eines Katalysators, umsetzt.

6. Schädlingsbekämpfungsmittel, gekennzeichnet durch einen Gehalt an mindestens einem 2,5-disubstituiertem 1,3,4-Thiadiazol-Derivat der Formel (I) nach den Ansprüchen 1 und 5.

7. Verwendung von 2,5-disubstituierten 1,3,4-Thiadiazol-Derivaten der Formel (I) nach den Ansprüchen 1 und 5 zur Bekämpfung von Schädlingen.

8. Verfahren zur Bekämpfung von Schädlingen, dadurch gekennzeichnet, daß man ein 2,5-disubstituiertes 1,3,4-Thiadiazol-Derivat der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man 2,5-disubstituierte 1,3,4-Thiadiazol-Derivate der Formel (I) nach den Ansprüchen 1 und 5 mit Streckmitteln und/oder oberflächenaktiven Mitteln vermischt.